# EUROPEAN PATENT APPLICATION

(11) **EP 4 563 586 A1**
(43) Date of publication of application: **04.06.2025**
(21) Application number: 23307078.8
(22) Date of filing: 28.11.2023
(51) Int. Cl.: C07K 7/08, A61K 38/00, C07K 14/47, C12N 9/00

(54) **NEW INHIBITORS OF LRRK2/PP1 INTERACTION**

(71) Applicant: Université Paris Cité, 75006 Paris (FR); Institut National de la Santé et de la Recherche Médicale, 75013 Paris (FR)
(72) Inventor: REBOLLO, Angelita, 75015 Paris (FR); ARON-BADIN, Romina, 92260 Fontenay-aux-Roses (FR); TEESALU, Tambet, 50090 Tartu (EE); LOISEL, Severine, 29238 Brest (FR)
(74) Representative: A.P.I. Conseil

(57) **Abstract**

The present invention provides new inhibitors of LRRK2/PP1 interaction able to pass through the Blain Blood Barrier and to target the brain. The present invention relates to these inhibitors for use as medicament and more particularly to methods and pharmaceutical compositions for the treatment of neurodegenerative disorders, more particularly α-synucleinopathies.

## Description

### FIELD OF THE INVENTION

The present invention provides new inhibitors of LRRK2/PP1 interaction which are able to efficiently pass through the BBB. The present invention relates to these inhibitors for use as medicament and more particularly to methods and pharmaceutical compositions related to the treatment of neurodegenerative disorders, more particularly α-synucleinopathies.

### BACKGROUND OF THE INVENTION

A common feature of neurodegenerative disorder is the loss of neuronal processes as neurite outgrowth and neuronal cell viability. Mutations in LRRK2 have been shown to induce reduction in neurite length and branching, tau aggregates formation and ultimately to lead to neuronal cell apoptosis (Mac Leod *et al.* 2006). Some have found that LRRK2 appears to be present in neuronal and glial inclusions in several neurodegenerative disorders, leading to the hypothesis that a common link may exist in the pathogenesis of these disorders (Miklossy *et al.,* 2006).

More specifically, mutations in the gene encoding LRRK2 are known to be responsible for the genetic forms of Parkinson's disease (PD). However, these mutations are not systematically associated with the development of the disease, factors preventing the development of the disease may be present in subjects carrying these mutations. Nevertheless, a significant proportion of apparently isolated cases of Parkinson's disease originates from a dominant mutation of the LRRK2 gene, resulting in the substitution G2019S: in North Africa it is present in 37% of familial PD cases. This mutation was also found in 41% of apparently isolated cases of PD in subjects of North African origin. Such large proportions of mutants in cases of parkinsonism are also observed in other specific populations. Highlighting even more the importance of LRRK2 in PD, the literature reports that the clinical symptoms associated with LRRK2 mutations associated with Parkinson's disease can not be distinguished from those of sporadic cases.

The G2019S mutation, like other mutations responsible for the autosomal transmission of the disease, is linked to a hyperactivation of the kinase activity (autophosphorylation) of LRRK2 and this is why there are some inhibitors of LRRK2 kinase activity which are currently in clinical testing. However, literature mentions side effects for inhibitors as altered vesicular trafficking and lysosomal function, mitochondrial dysfunction, and lung and kidney abnormalities.

Some PD-related mutations are also found associated with a reduced phosphorylation of LRRK2, particularly in a cluster of serines (binding site P14 3 3), and are related to a change in cell localization of the protein which varies as a function of cell type and which is also found altered in PD patients. Phosphorylation sites are also related to the kinase activity of the protein because they are dephosphorylated in the presence of LRRK2 inhibitors. The serine/threonine protein phosphatase 1 (PP1) is a regulator of the LRRK2 phosphorylation cycle and is responsible for LRRK2 dephosphorylation observed in PD mutant LRRK2 and after LRRK2 kinase inhibition.

Accordingly, inhibition of LRRK2/PP1 interaction is identified as a valuable therapeutic strategy in treating neurological disorders, more particularly α-synucleinopathies as Parkinson's disease, which yet remain an unmet medical need.

The interacting region of LRRK2 with PP1 and inhibiting peptides derived thereof are disclosed in patent WO2020193441. Yet, there is still a need in providing further LRRK2/PP1 interaction inhibiting peptides and in improving their stability and ability to efficiently target brain.

### SUMMARY OF THE INVENTION

invention is thus related to new peptides that constitute valuable candidates for treating neurological disorders.

According to a **first object,** the present invention relates to a peptide inhibitor of LRRK2/PP1 interaction of following structure:
Nt -Cargo - Linker - Shuttle- Ct, wherein :
- "Shuttle" moiety is defined as a peptide of sequence of SEQ ID NO:1,
- "Linker" moiety is selected from Amino-PEG4-alcohol, Amino-PEG6-alcohol, Amino-PEG8-alcohol, Amino-PEG12-alcohol, Amino-PEG8-acid, Amino-PEG12-acid, Amino-PEG24-acid, 6-aminohexanoic acid, SEQ ID NO:6, SEQ ID NO:33, SEQ ID NO:34, SEQ ID NO:35, SEQ ID NO:36, SEQ ID NO:37, SEQ ID NO:38 SEQ ID NO:39.
- "Cargo" moiety is selected from :
   i) a peptide selected from a peptide of sequence of SEQ ID NO:2, SEQ ID NO:3 or SEQ ID NO:4, or a conservative variant thereof,
   ii) a peptide of a sequence of at least 7 amino acids in the region ranging from the residue at position 1701 to the amino acid residue at position 1715 of SEQ ID NO:5, or a conservative variant thereof.

As exemplified, said peptide inhibitor of said structure are found effective in targeting passing through BBB and targeting neuronal cells, whereas others are not.

According to other optional features of the peptide inhibitor according to the invention, it can optionally include one or more of the following characteristics alone or in combination:
- the "Cargo" moiety is selected from : SEQ ID NO:7, SEQ ID NO:8, SEQ ID NO:9, SEQ ID NO:10, SEQ ID NO:11, SEQ ID NO:12, SEQ ID NO:13 , SEQ ID NO:14 or a conservative variant thereof.
- the "Linker" moiety is selected from the tripeptide of SEQ ID NO:6 or 6-aminohexanoic acid.
- the "Linker" moiety is selected 6-aminohexanoic acid and the "Cargo" moiety is SEQ ID NO:13 .
- the "Linker" is the tripeptide of sequence SEQ ID NO:6.

According to **a second object** the present invention also relates to a nucleic acid molecule encoding for the peptide according to the present invention, wherein the linker is a peptide.

According to a particular embodiment, the present invention also relates to a vector which comprises the nucleic acid according to the present invention.

According to **a third object,** the present invention also relates to a host cell transformed with the nucleic acid molecule according to the present invention.

Such kind of molecule encoding a peptide inhibitor of the invention can be of use in the biotechnological field (as a research tool or for producing the inhibitor) as well as in the medical field.

According to **a fourth object,** the present invention also relates to an antibody or aptamer which specifically binds to a peptide selected from a peptide of sequence of SEQ ID NO:2, SEQ ID NO:3 or of SEQ ID NO:4. These peptides are region of PP1 and are found to effectively inhibit LRRK2/PP1 interaction.

In a **fifth object,** and in accordance with the property of the above described objects to interfere with LRRK2/PP1 interaction the present invention relates to an agent selected from :
- a peptide inhibitor of LRRK2/PP1 interaction of structure Nt -Cargo - Linker - Shuttle- Ct,as defined above,
- a nucleic acid as defined above, or
- an aptamer or an antibody of claim 9 which specifically binds to a peptide selected from a peptide of sequence of SEQ ID NO:2, SEQ ID NO:3 or of SEQ ID NO:4 as defined above,
for use as a medicament.

Indeed, PP1 is known to form complexes with LRRK2 and to regulate the activity of this latter. Beside dysregulation of LRRK2 is clearly linked to pathologies like α-synucleinopathies.

Accordingly, in a particular embodiment, the invention relates to any of the agents as described above for use in the treatment of a neurodegenerative disorder. In a particular embodiment, said neurodegenerative disorder is an α-synucleinopathy, preferably selected from Parkinson's disease (PD), dementia with Lewy bodies (DLB), and multiple system atrophy (MSA).

According to **a further object,** the present invention also relates to an isolated peptide inhibitor of LRRK2/PP1 comprising a peptide of sequence of SEQ ID NO:2, SEQ ID NO:3 or SEQ ID NO:4, or a conservative variant thereof.

### FIGURES

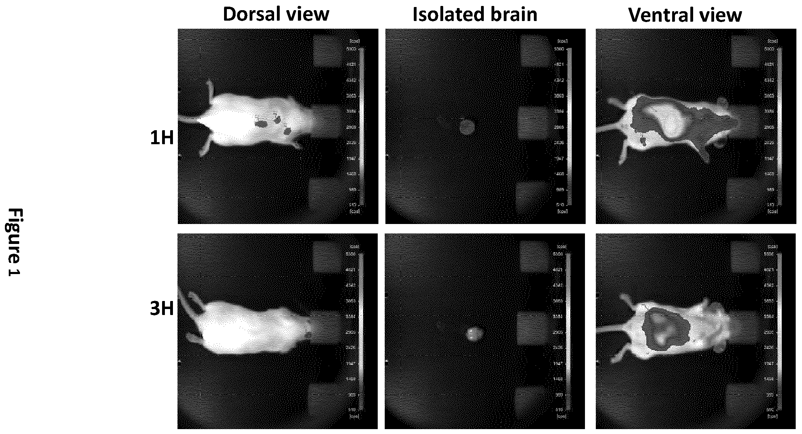

- **Figure 1** : *In vivo* images of a mouse injected with Cy7 labelled peptide 1 (retroorbital injection, 5mg/kg). Images were taken at 1, and 3 hours from injection. The scale bar on the right shows the intensity of the fluorescence. Red represents higher intensity and purple low intensity. Dorsal and Ventral are shown as well as images of a brain of an injected animal isolated at the corresponding time.

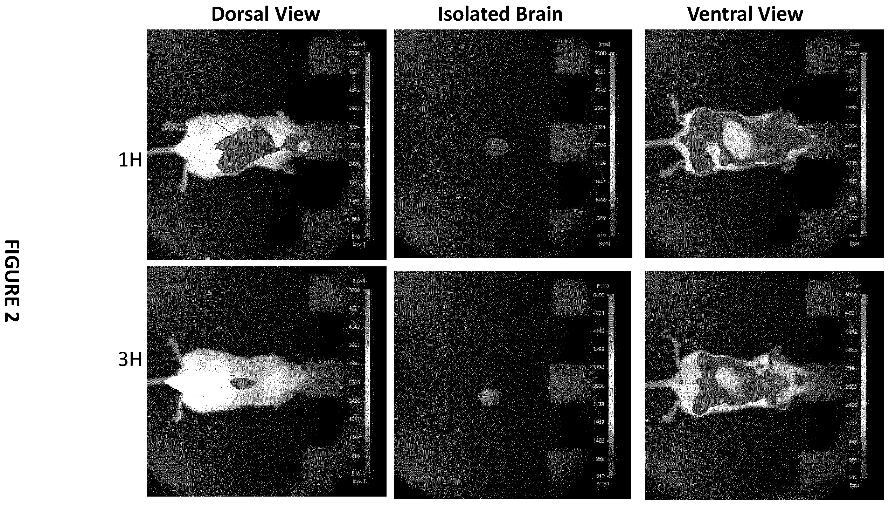

- **Figure 2** : *In vivo* images of a mouse injected with Cy7 labelled peptide 2 (retroorbital injection, 5mg/kg). Images were taken at 1, 3 hours from injection. The scale bar on the right shows the intensity of the fluorescence. Red represents higher intensity and purple low intensity. Dorsal and Ventral are shown as well as images of a brain of an injected animal isolated at the corresponding time.

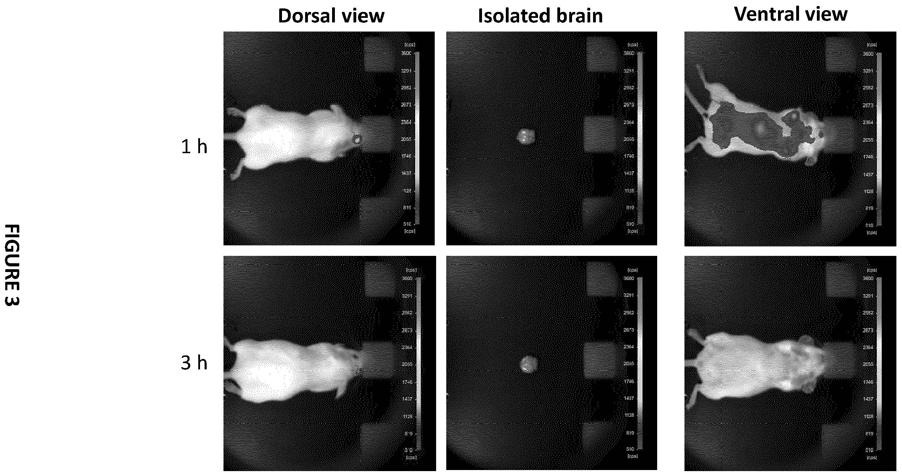

- **Figure 3** : *In* vivo images of a mouse injected with a Cy5 labelled control peptide made of a Tumor penetrating peptide Lin TT1 shuttle peptide associated in Nter- to the peptide of SEQ ID NO:13 (retroorbital injection, 5mg/kg). Images were taken at 1 and 3 hours from injection. The scale bar on the right shows the intensity of the fluorescence. Red represents higher intensity and purple low intensity. Dorsal and Ventral are shown as well as images of a brain of an injected animal isolated at the corresponding time.

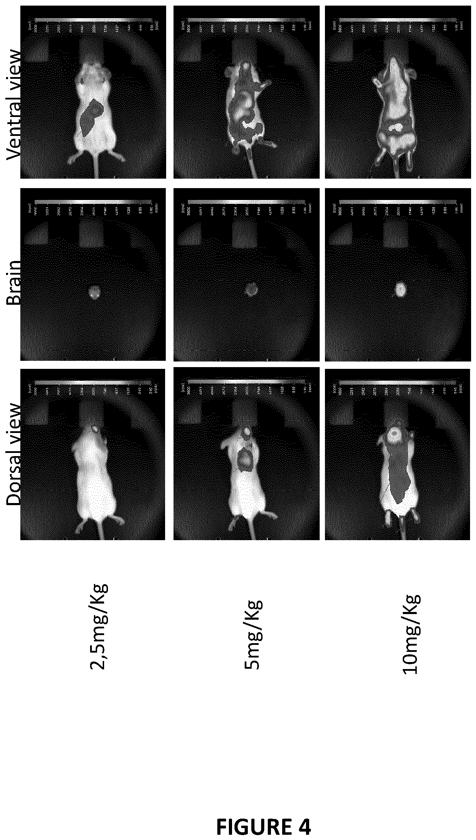

- **Figure 4** : Concentration kinetic of Cy7-labelled peptide SEQ ID NO:15 in a mouse injected with 2.5, 5 and 10 mg/kg Cy7-labelled peptide SEQ ID NO:15 (T=1 hour from injection). Red represents higher intensity and purple low intensity. Dorsal and Ventral are shown as well as images of a brain of an injected animal isolated at the corresponding time.

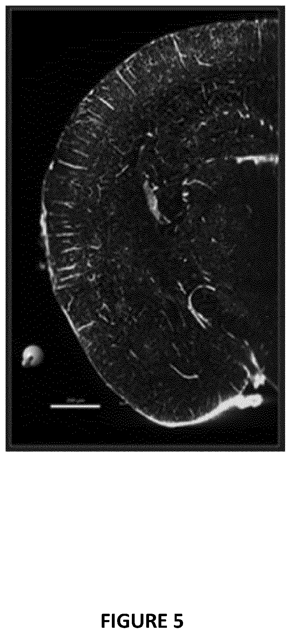

- **Figure 5** : Immunohistochemistry labelling of mouse embryo midbrain with a peptide of the invention.

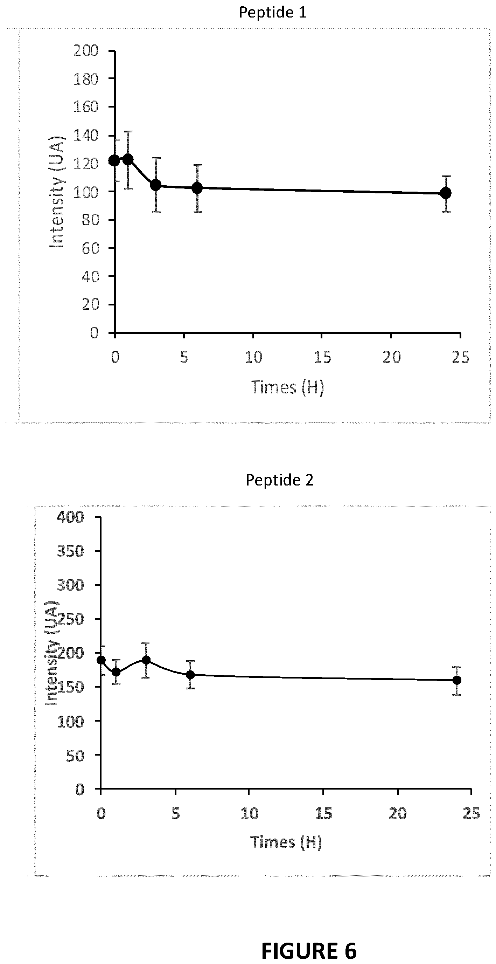

- **Figure 6** Kinetic of degradation of peptides of the invention (100 µM) in human serum, estimated from Maldi-Toff quantitation, arbitrary units.

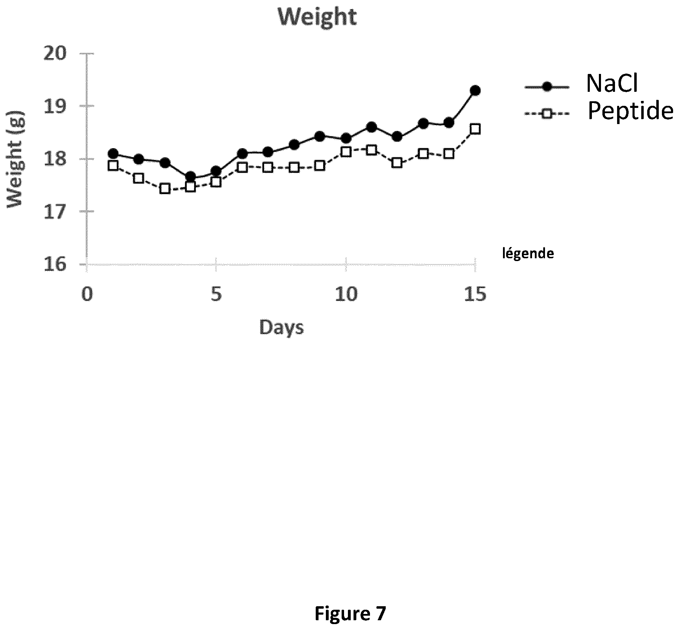

- **Figure 7** : Evolution of bodyweight of mice administered IP for two weeks, 5 days per week with a dose of 5 mg/kg of peptide 1.

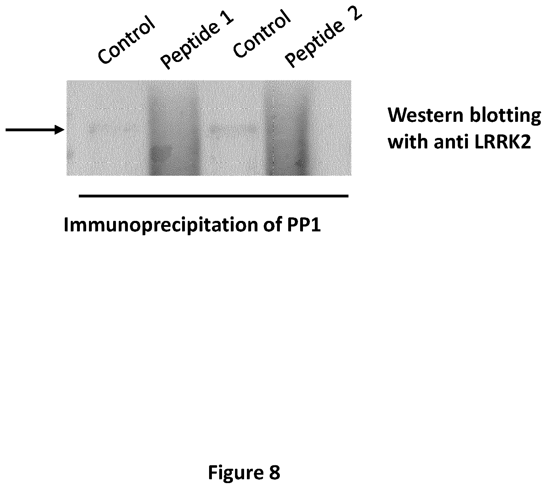

- **Figure 8****:** *In vitro* competition assay of LRRK2/PP1 interaction with peptides according to the invention. Western blotting using LRRK2 antibody to detect LRRK2 in PP1 immunoprecipitates after incubation with peptides 1 or 2; control : no competed immunoprecipitates. Immunoprecipitates were washed and immunoblotted with an anti-LRRK2 antibody.

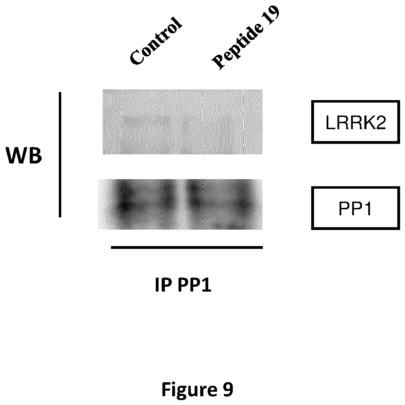

- **Figure 9****:** *In vitro* competition assay of LRRK2/PP1 interaction with peptides according to the invention. Western blotting using anti LRRK2 or PP1 antibody to detect LRRK2 and PP1 respectively in PP1 immunoprecipitates after incubation with peptide 19 control : no competed immunoprecipitates. Immunoprecipitates were washed and immunoblotted with an anti-LRRK2 antibody.

### DETAILED DESCRIPTION OF THE INVENTION

In the present invention, the inventors investigated the interaction of PP1 and LRRK2 and developed new peptides that effectively inhibit PP1/LRRK2 interaction while being able to cross the Blood Brain Barrier (BBB) to ultimately target brain neuronal cells, thereby providing valuable candidates for the treatment of neurological diseases linked to LRRK2 kinase activity.

### Polypeptides of the invention

The present invention relates to isolated, synthetic or recombinant polypeptides which are inhibitors of LRRK2/PP1 interaction. The invention relates also to polypeptides which are found resistant to protease degradation, non-toxic and able to cross BBB to target neuronal cells, wherein the inhibition of PP1/LRRK2 is thought to provide a therapeutic effect. Indeed, as mentioned above, mutation in LRRK2 are found associated to PD and it has been shown that exposition to peptides capable of *in vitro* inhibiting PP1/LRRK2 promotes neurites outgrowth.

Except otherwise stated, all peptide sequences are displayed following the writing convention for peptide sequences, from the N-terminal (Nt) end to the C-terminal (Ct) end.

As used herein the term "LRRK2" has its general meaning in the art and refers to Leucine-rich repeat kinase 2, also known as dardarin is an enzyme that in humans is encoded by the PARK8 gene. LRRK2 is a member of the leucine-rich repeat kinase family. Variants of this gene are associated with an increased risk of Parkinson's disease and also Crohn's disease. An exemplary human polypeptide sequence of LRRK2 is SEQ ID NO:5. Of course, based on the teachings of this disclosure, the skilled in the art is able to identify within the amino acid sequence of homologous protein to LRRK2 of mammals the corresponding region of interaction of LRRK2 and PP1.

SEQ ID NO:5

MASGSCQGCEEDEETLKKLIVRLNNVQEGKQIETLVQILEDLLVFTYSEHASKLFQGK
NIHVPLLIVLDSYMRVASVQQVGWSLLCKLIEVCPGTMQSLMGPQDVGNDWEVLGV
HQLILKMLTVHNASVNLSVIGLKTLDLLLTSGKITLLILDEESDIFMLIFDAMHSFPANDE
VQKLGCKALHVLFERVSEEQLTEFVENKDYMILLSASTNFKDEEEIVLHVLHCLHSLAI
PCNNVEVLMSGNVRCYNIVVEAMKAFPMSERIQEVSCCLLHRLTLGNFFNILVLNEV
HEFVVKAVQQYPENAALQISALSCLALLTETIFLNQDLEEKNENQENDDEGEEDKLF
WLEACYKALTWHRKNKHVQEAACWALNNLLMYQNSLHEKIGDEDGHFPAHREVML
SMLMHSSSKEVFQASANALSTLLEQNVNFRKILLSKGIHLNVLELMQKHIHSPEVAES
GCKMLNHLFEGSNTSLDIMAAVVPKILTVMKRHETSLPVQLEALRAILHFIVPGMPEE
SREDTEFHHKLNMVKKQCFKNDIHKLVLAALNRFIGNPGIQKCGLKVISSIVHFPDAL
EMLSLEGAMDSVLHTLQMYPDDQEIQCLGLSLIGYLITKKNVFIGTGHLLAKILVSSLY
RFKDVAEIQTKGFQTILAILKLSASFSKLLVHHSFDLVIFHQMSSNIMEQKDQQFLNLC
CKCFAKVAMDDYLKNVMLERACDQNNSIMVECLLLLGADANQAKEGSSLICQVCEK
ESSPKLVELLLNSGSREQDVRKALTISIGKGDSQIISLLLRRLALDVANNSICLGGFCIG
KVEPSWLGPLFPDKTSNLRKQTNIASTLARMVIRYQMKSAVEEGTASGSDGNFSED
VLSKFDEWTFIPDSSMDSVFAQSDDLDSEGSEGSFLVKKKSNSISVGEFYRDAVLQ
RCSPNLQRHSNSLGPIFDHEDLLKRKRKILSSDDSLRSSKLQSHMRHSDSISSLASE
REYITSLDLSANELRDIDALSQKCCISVHLEHLEKLELHQNALTSFPQQLCETLKSLTH
LDLHSNKFTSFPSYLLKMSCIANLDVSRNDIGPSVVLDPTVKCPTLKQFNLSYNQLSF
VPENLTDVVEKLEQLILEGNKISGICSPLRLKELKILNLSKNHISSLSENFLEACPKVES
FSARMNFLAAMPFLPPSMTILKLSQNKFSCIPEAILNLPHLRSLDMSSNDIQYLPGPA
HWKSLNLRELLFSHNQISILDLSEKAYLWSRVEKLHLSHNKLKEIPPEIGCLENLTSLD
VSYNLELRSFPNEMGKLSKIWDLPLDELHLNFDFKHIGCKAKDIIRFLQQRLKKAVPY
NRMKLMIVGNTGSGKTTLLQQLMKTKKSDLGMQSATVGIDVKDWPIQIRDKRKRDL
VLNVWDFAGREEFYSTHPHFMTQRALYLAVYDLSKGQAEVDAMKPWLFNIKARASS
SPVILVGTHLDVSDEKQRKACMSKITKELLNKRGFPAIRDYHFVNATEESDALAKLRK
TIINESLNFKIRDQLVVGQLIPDCYVELEKIILSERKNVPIEFPVIDRKRLLQLVRENQLQ
LDENELPHAVHFLNESGVLLHFQDPALQLSDLYFVEPKWLCKIMAQILTVKVEGCPK
HPKGIISRRDVEKFLSKKRKFPKNYMSQYFKLLEKFQIALPIGEEYLLVPSSLSDHRPV
IELPHCENSEIIIRLYEMPYFPMGFWSRLINRLLEISPYMLSGRERALRPNRMYWRQG
IYLNWSPEAYCLVGSEVLDNHPESFLKITVPSCRKGCILLGQVVDHIDSLMEEWFPGL
LEIDICGEGETLLKKWALYSFNDGEEHQKILLDDLMKKAEEGDLLVNPDQPRLTIPISQ
IAPDLILADLPRNIMLNNDELEFEQAPEFLLGDGSFGSVYRAAYEGEEVAVKIFNKHT

SLRLLRQELVVLCHLHHPSLISLLAAGIRPRMLVMELASKGSLDRLLQQDKASLTRTL
QHRIALHVADGLRYLHSAMIIYRDLKPHNVLLFTLYPNAAIIAKIADYGIAQYCCRMGIK
TSEGTPGFRAPEVARGNVIYNQQADVYSFGLLLYDILTTGGRIVEGLKFPNEFDELEI
QGKLPDPVKEYGCAPWPMVEKLIKQCLKENPQERPTSAQVFDILNSAELVCLTRRIL
LPKNVIVECMVATHHNSRNASIWLGCGHTDRGQLSFLDLNTEGYTSEEVADSRILCL
ALVHLPVEKESWIVSGTQSGTLLVINTEDGKKRHTLEKMTDSVTCLYCNSFSKQSKQ
KNFLLVGTADGKLAIFEDKTVKLKGAAPLKILNIGNVSTPLMCLSESTNSTERNVMWG
GCGTKIFSFSNDFTIQKLIETRTSQLFSYAAFSDSNIITVVVDTALYIAKQNSPVVEVW
DKKTEKLCGLIDCVHFLREVMVKENKESKHKMSYSGRVKTLCLQKNTALWIGTGGG
HILLLDLSTRRLIRVIYNFCNSVRVMMTAQLGSLKNVMLVLGYNRKNTEGTQKQKEIQ
SCLTVWDINLPHEVQNLEKHIEVRKELAEKMRRTSVE.

As used herein the term "PP1" for Protein Phosphatase 1 has its general meaning in the art, that is serine/threonine protein phosphatase 1. PP1 is one of the most ubiquitous and abundant serine/threonine phosphatases in eukaryotic cells. Protein Phosphatases are implicated in the regulation of various essential cellular functions: PP1 is found to play pivotal role in a wide variety of physiological and molecular processes as glycogen metabolism, muscle contraction, cell progression, neuronal activities, apoptosis etc .... and consequently, suspected to be implicated in numerous complex diseases. This versatility can be explained by the nearly 200 validated interactors in vertebrates (among which LRRK2). An exemplary human polypeptide sequence of PP1 is SEQ ID NO:17. Of course, based on the teachings of this disclosure, the skilled in the art is able to identify within the amino acid sequence of homologous protein to LRRK2 of mammals the corresponding region of interaction of LRRK2 and PP1.

SEQ ID NO:17

MSDSEKLNLDSIIGRLLEVQGSRPGKNVQLTENEIRGLCLKSREIFLSQPILLELEAPL

KICGDIHGQYYDLLRLFEYGGFPPESNYLFLGDYVDRGKQSLETICLLLAYKIKYPEN

FFLLRGNHECASINRIYGFYDECKRRYNIKLWKTFTDCFNCLPIAAIVDEKIFCCHGGL

SPDLQSMEQIRRIMRPTDVPDQGLLCDLLWSDPDKDVQGWGENDRGVSFTFGAEV

VAKFLHKHDLDLICRAHQVVEDGYEFFAKRQLVTLFSAPNYCGEFDNAGAMMSVDE

TLMCSFQILKPADKNKGKYGQFSGLNPGGRPITPPRNSAKAKK.

As used herein the terms "polypeptide", "peptide" or "peptide moiety" are used interchangeably. They designate a chain of amino acid monomers linked by peptides bonds. Of course, in some instance, specifically when using analogs to some aminoacids or linker molecules which are commonly used in peptide derivative pharmaceutical treatments, said bond can be different from the usual peptide bond. Also, said peptide or polypeptide can be linear or cyclic. In some embodiment peptide of the invention is cyclic as cyclic peptides tend to be extremely resistant to the processes of degradation which is of particular interest for the sake of developing medicaments.

A "conservative variant", when related to any peptide, polypeptide or peptide moiety disclosed herein, is meant to exhibit a sequence identity of typically at least about 60%, preferably at least about 70%, more preferably at least about 90%, more preferably at least about 95% with said peptide polypeptide or peptide moiety. More particularly, a conservative variant or said peptide, polypeptide or peptide moiety is thought to effectively inhibit interaction in an *in vitro* assay such as a western blotting assay or an immunoprecipitation assay as disclosed in the experimental section, more particularly said inhibition is at least of 60%, at least of 70%, at least of 80%, at least of 90%, even at least 95% or even more, of the inhibition level of peptide SEQ ID NO : 13 in the corresponding assay.

As used herein the term "inhibitor of LRRK2/PP1 interaction" refers to a compound that is able to inhibit the interaction of LRRK2 with PP1. As demonstrated in the example section, polypeptides of the invention are able to inhibit LRRK2/PP1 interaction. These polypeptides are thought to compete with either LRRK2 or PP1 to impede the interaction on these two proteins. Based on structural analyses, Inventors have been able to delineate the "minimum" peptides, inhibitors of LRRK2/PP1 interaction consisting of a peptide fragment of sequence SEQ ID NO:5, or in a conservative variant thereof, or in a peptide fragment of SEQ ID NO:17, or a conservative variant thereof. Though of being of an evident interest as such, these minimum peptides because of their short length constitute convenient cargo moieties to be addressed to brain neuronal cells to inhibit pathological LRRK2/PP1 interaction in diseased cells. Todo so, they need to be coupled to shuttle vector in order to improve their bioavailability to the brain. Nevertheless, producing chimeric polypeptides, that are stable, conveniently addressed to the desired body compartment (here the brain), while retaining their biological activity remains challenging.

Inventors have been able elaborate recombinant artificial peptides which are peptides inhibitors of LRRK2/PP1 interaction and which display the following structure:
Nt -Cargo - Linker - Shuttle- Ct.

Inventors have surprisingly found that other structures such as Nt -Shuttle - Linker - Cargo- Ct are not effective in passing through the BBB and/or targeting neuronal cells and/or inhibiting LRRK2/PP1 interaction.

In the peptides inhibitors of LRRK2/PP1 interaction according to the invention, "shuttle" moiety refers to a peptide moiety of sequence of SEQ ID NO:1, which is found effective in allowing the inhibitors of LRRK2/PP1 interaction to pass the BBB and in specifically addressing neuronal cell. This shuttle is of shortest in regard to other shuttle peptides design to pass the BBB, e.g. angiopep2, and, further to addressing the peptides to neuronal cells, thus of particular interest in e.g. terms of size, cost of synthesis. A low hindrance is expected to less interfering with the activity of the cargo moiety than bigger shuttle moieties of the art.
SEQ ID NO:1 : SRRVISRAKLAAAL.

Without wishing to be bound by any theory, peptide of sequence SEQ ID NO:1 has been shown to specifically bound reelin (encoded by gene *RELN*, e.g. Gene ID: 5649 for human protein), a glycoprotein from extracellular matrix of neuron that is thought to play a role in synapse plasticity. It has been found that, *in vitro,* said peptide is efficiently internalized within neuronal cells (PC12 cell line).

In the inhibitors of LRRK2/PP1 interaction according to the invention a "Linker" moiety ensures the flexibility of the molecule to allow the cargo moiety and shuttle moiety to freely exert their function independently. The linker moiety can be selected from Amino-PEG4-alcohol, Amino-PEG6-alcohol, Amino-PEG8-alcohol, Amino-PEG12-alcohol, Amino-PEG8-acid, Amino-PEG12-acid, Amino-PEG24-acid, 6-aminohexanoic acid, tripeptide GGS (SEQ ID NO:6), tripeptide GGG (SEQ ID NO:33), tripeptide AAN (SEQ ID NO:34), dipeptide GG (SEQ ID NO:35), dipeptide FK (SEQ ID NO:36), dipeptide VA (SEQ ID NO:37), dipeptide VV (SEQ ID NO:38), or dipeptide VG (SEQ ID NO:39).

In a particular embodiment said linker moiety is a peptide of SEQ ID NO:6 : GGS.

In another particular embodiment, which is preferred, said linker is 6-aminohexanoic acid (CAS number 60-32-2). In an embodiment, the cargo moiety can be bound, at its C-terminal end, to the amino group of the 6-aminohexanoic acid whereas the hydroxyl group of the 6-aminohexanoic acid is linked to the N-terminal end of the shuttle moiety.

In an embodiment of the peptide inhibitors of LRRK2/PP1 interaction according to the invention, the "cargo" moiety can comprise peptides of SEQ ID NO:2 : VQGSRPGKNVQLTENE, SEQ ID NO:3 : AEVVAKFLHKHDLDLICRAH or of SEQ ID NO:4 : NPGGRPITPPRNSAKAKK or a conservative variant thereof.

Without wishing to be bound by any theory, these peptides are thought to be implicated in the interaction region of PP1 protein with LRRK2, and are found by the inventors to inhibit LRRK2/PP1 interaction *in vitro.*

In another embodiment of the peptide inhibitors of LRRK2/PP1 interaction according to the invention, the "cargo" moiety can comprise a peptide of a sequence of at least 7 amino acids in the region ranging from the residue at position 1701 to the amino acid residue at position 1715 of SEQ ID NO:5, or a conservative variant thereof.

In one embodiment said cargo moiety of the inhibitor of LRRK2/PP1 interaction of the invention is a polypeptide which i) consists of a fragment of peptide of SEQ ID NO:5 which comprises at least the 7 consecutive amino acids ranging from amino acid residue at position 1709 to amino acid at position 1715 of said SEQ ID NO:5, and ii) consists of a sequence of amino acids in the region ranging from the residue at position 1701 to the amino acid residue at position 1718 of SEQ ID NO:5, as known from WO2020193441. In a particular embodiment said cargo moiety has at least 70% of identity with the sequence of at least 7 amino acids ranging from the amino acid residue at position 1709 to the amino acid residue at position 1715. In another particular embodiment, said cargo moiety which consists of a fragment of peptide of SEQ ID NO:5 and which comprises at least the 7 consecutive amino acids ranging from amino acid residue at position 1709 to amino acid at position 1715 of said SEQ ID NO:5, further comprises the amino acid residues W1705, S1706, R1707, 11709, R1711, L1712, L1713, E1714. In more particular embodiment said cargo moiety is 18 amino acids long.

In another embodiment a cardo moiety of the peptide inhibitor of LRRK2/PP1 interaction of the invention consists of a sequence of at least 7 amino acids in the region ranging from the residue at position 1701 to the amino acid residue at position 1718 of SEQ ID NO:5. In more particular embodiments a cardo moiety consists of a sequence of 8, 9, 10, 11, 12, 13, 14, 15, 16, 17 or even 18 amino acids in the region ranging from the residue at position 1701 to the amino acid residue at position 1718 of SEQ ID NO:5.

In another embodiment said peptide inhibitor of LRRK2/PP1 interaction, which consist in a fragment of SEQ ID NO:5 is selected from :
SEQ ID NO:7 : INRLLEISPY,
SEQ ID NO:8 : LINRLLEISPY,
SEQ ID NO:9 : SRLINRLLEISPY,
SEQ ID NO:10 : PMGFWSRLI,
SEQ ID NO:11 : GFWSRLINRLLEISPY,
SEQ ID NO:12 : PMGFWSRLINRLLEISPY,
SEQ ID NO:13 : GFWSRLINRLLEI, or
SEQ ID NO:14: PMGFWSRLINRLLEI,
or a conservative variant thereof.

In a preferred embodiment, in the peptides inhibitors of LRRK2/PP1 interaction according to the invention, the shuttle is peptide of sequence SEQ ID NO:1 and the linker is the tripeptide of sequence SEQ ID NO:6; in another preferred embodiment, in the peptides inhibitors of LRRK2/PP1 interaction according to the invention, the shuttle is peptide of sequence SEQ ID NO:1 and the linker is 6-aminohexanoic acid.

In a particularly preferred embodiment, in a peptide inhibitor of LRRK2/PP1 interaction the shuttle moiety is peptide of SEQ ID NO:1, the linker is 6-aminohexanoic acid and the cargo moiety is of sequence SEQ ID NO:13.

### Peptides of sequences SEQ ID NO:2, SEQ ID NO:3 or of SEQ ID NO:4

Peptides of sequence SEQ ID NO:2, SEQ ID NO:3 or of SEQ ID NO:4 correspond to isolated fragments of PP1 which interact with LRRK2 when LRRK2 and PP1 interact. They have been found to bind to LRRK2 *in vitro.* These purified peptides are found by the inventors to inhibit LRRK2/PP1 interaction *in vitro.*

A second object of the invention then relates to these purified isolated peptides of sequence SEQ ID NO:2, SEQ ID NO:3 or of SEQ ID NO:4, or conservative variant thereof, which constitutes valuable research tools and medicament candidates.

### Nucleic acid encoding peptides inhibitor of LRRK2/PP1 interaction.

A further object of the invention relates to a nucleic acid sequence encoding for a peptide inhibitor of LRRK2/PP1 interaction according to the invention described previously. In this embodiment in said polypeptide inhibitor of LRRK2/PP1 interaction, the linker moiety is a peptide, preferably an oligopeptide.

As used herein, a sequence "encoding" an expression product, such as a RNA, polypeptide, protein, or enzyme, is a nucleotide sequence that, when expressed, results in the production of that RNA or corresponding polypeptide i.e., the nucleotide sequence encodes an amino acid sequence for that polypeptide. Typically, said nucleic acid is a DNA or RNA molecule, which may be included in a suitable vector, such as a plasmid, cosmid, episome, artificial chromosome, phage or viral vector.

So, a further object of the present invention relates to a vector and an expression cassette in which a nucleic acid molecule encoding for a polypeptide of the invention is associated with suitable elements for controlling transcription (in particular promoter, enhancer and, optionally, terminator) and, optionally translation, and also the recombinant vectors into which a nucleic acid molecule in accordance with the invention is inserted. These recombinant vectors may, for example, be cloning vectors, or expression vectors.

As used herein, the terms "vector", "cloning vector" and "expression vector" mean the vehicle by which a DNA or RNA sequence encoding a peptide according to the invention can be introduced into a host cell, so as to transform the host and promote expression (e.g. transcription and translation) of the introduced sequence.

Any expression vector for animal cell can be used and will be easily identified by the skilled in the art.

A further aspect of the invention relates to a host cell comprising a nucleic acid molecule encoding for a polypeptide according to the invention or a vector according to the invention. These host cells can be used for example to produce peptides of the invention, to amplify genetic material or for gene therapy purposes. In particular, a subject of the present invention is a prokaryotic or eukaryotic host cell genetically transformed with at least one nucleic acid molecule or vector according to the invention.

The construction of expression vectors in accordance with the invention, and the transformation of the host cells can be carried out using conventional molecular biology techniques well known from those skilled in the art.

More particularly, nucleic acid sequences encoding said peptides can be delivered into cells of interest by any suitable mean, in e.g., inter alia viral vectors, in order to allow the expression of said peptides into targeted cells thereby rendering useless fusion to vectorization peptides. Gene delivery viral vectors useful in the practice of the present invention can be constructed utilizing methodologies well known in the art of molecular biology. Typically, viral vectors carrying transgenes (in other words gene encoding peptide of the invention) are assembled from polynucleotides encoding the transgene, suitable regulatory elements and elements necessary for production of viral proteins which mediate cell transduction.

The terms "Gene transfer" or "gene delivery" refer to methods or systems for reliably inserting foreign DNA into host cells. Such methods can result in transient expression of non-integrated transferred DNA, extrachromosomal replication and expression of transferred replicons (e. g., episomes), or integration of transferred genetic material into the genomic DNA of host cells.

Examples of viral vector include adenoviral, retroviral, herpesvirus and adeno-associated virus (AAV) vectors.

Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

In a preferred embodiment, adeno-associated viral (AAV) vectors are employed. By an "AAV vector" is meant a vector derived from an adeno-associated virus serotype, including without limitation, AAV-1, AAV-2, AAV-3, AAV-4, AAV-5, AAV6, etc. AAV vectors can have one or more of the AAV wild-type genes deleted in whole or part, preferably the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e. g., functional ITRs) of the virus. The ITRs need not be the wild-type nucleotide sequences, and may be altered, e. g., by the insertion, deletion or substitution of nucleotides, so long as the sequences provide for functional rescue, replication and packaging. AAV expression vectors are constructed using known techniques to at least provide as operatively linked components in the direction of transcription, control elements including a transcriptional initiation region, the DNA of interest (*i.e*. encoding a peptide of the invention) and a transcriptional termination region.

The control elements are selected to be functional in a mammalian cell. The resulting construct which contains the operatively linked components is bounded (5' and 3') with functional AAV ITR sequences. By "adeno-associated virus inverted terminal repeats " or "AAV ITRs" is meant the art-recognized regions found at each end of the AAV genome which function together in cis as origins of DNA replication and as packaging signals for the virus. AAV ITRs, together with the AAV rep coding region, provide for the efficient excision and rescue from, and integration of a nucleotide sequence interposed between two flanking ITRs into a mammalian cell genome. The nucleotide sequences of AAV ITR regions are known. See, e. g., Kotin,1994 ; Berns, KI "Parvoviridae and their Replication" in Fundamental Virology, 2nd Edition, (B. N. Fields and D. M. Knipe, eds. ) for the AAV-2 sequence. As used herein, an "AAV ITR" does not necessarily comprise the wild-type nucleotide sequence, but may be altered, e. g., by the insertion, deletion or substitution of nucleotides. Additionally, the AAV ITR may be derived from any of several AAV serotypes, including without limitation, AAV-1, AAV-2, AAV-3, AAV-4, AAV-5, AAV-6, etc. Furthermore, 5' and 3'ITRs which flank a selected nucleotide sequence in an AAV vector need not necessarily be identical or derived from the same AAV serotype or isolate, so long as they function as intended, i. e. to allow for excision and rescue of the sequence of interest from a host cell genome or vector, and to allow integration of the heterologous sequence into the recipient cell genome when AAV Rep gene products are present in the cell. Additionally, AAV ITRs may be derived from any of several AAV serotypes, including without limitation, AAV-1, AAV-2, AAV-3, AAV-4, AAV-5, AAV-6, etc. Furthermore, 5' and 3' ITRs which flank a selected nucleotide sequence in an AAV expression vector need not necessarily be identical or derived from the same AAV serotype or isolate, so long as they function as intended, i. e. to allow for excision and rescue of the sequence of interest from a host cell genome or vector, and to allow integration of the DNA molecule into the recipient cell genome when AAV Rep gene products are present in the cell.

Particularly preferred are vectors derived from AAV serotypes having tropism for and high transduction efficiencies in cells of the mammalian CNS, particularly neurons. A review and comparison of transduction efficiencies of different serotypes is provided in Davidson et al., 2000. In one preferred example, AAV-2 based vectors have been shown to direct long-term expression of transgenes in CNS, preferably transducing neurons. In other nonlimiting examples, preferred vectors include vectors derived from AAV-4 and AAV-5 serotypes, which have also been shown to transduce cells of the CNS (Davidson et al., supra).

The selected nucleotide sequence is operably linked to control elements that direct the transcription or expression thereof in the subject *in vivo.* Such control elements can comprise control sequences normally associated with the selected gene.

Alternatively, heterologous control sequences can be employed. Useful heterologous control sequences generally include those derived from sequences encoding mammalian or viral genes. Examples include, but are not limited to, the phophoglycerate kinase (PKG) promoter, the SV40 early promoter, mouse mammary tumor virus LTR promoter, adenovirus major late promoter (Ad MLP), a herpes simplex virus (HSV) promoter, a cytomegalovirus (CMV) promoter such as the CMV immediate early promoter region (CMVIE), Rous sarcoma virus (RSV) promoter, synthetic promoters, hybrid promoters, and the like. In addition, sequences derived from non-viral genes, such as the murine metallothionein gene, will also find use herein. Such promoter sequences are commercially available from, e. g., Stratagene (San Diego, CA). For purposes of the present invention, both heterologous promoters and other control elements, such as CNS-specific and inducible promoters, enhancers and the like, will be of particular use.

Examples of heterologous promoters include the CMV promoter. Examples of CNS specific promoters include those isolated from the genes from myelin basic protein (MBP), glial fibrillary acid protein (GFAP), and neuron specific enolase (NSE).

Examples of inducible promoters include DNA responsive elements for ecdysone, tetracycline.....

The AAV expression vector which harbors the DNA molecule encoding the peptides of the invention bounded by AAV ITRs, can be constructed by directly inserting the selected sequence(s) into an AAV genome which has had the major AAV open reading frames ("ORFs") excised therefrom. Other portions of the AAV genome can also be deleted, so long as a sufficient portion of the ITRs remain to allow for replication and packaging functions. Such constructs can be designed using techniques well known in the art. See, e. g., U. S. Patents Nos. 5,173, 414and 5,139, 941; International patent applications WO 92/01070 and WO 93/03769; Lebkowski et al., 1988 ; Vincent et al., 1990; Carter, 1992 ; Muzyczka, 1992 ; Kotin,1994; Shelling and Smith, 1994 ; and Zhou et al., 1994.) Alternatively, AAV ITRs can be excised from the viral genome or from an AAV vector containing the same and fused 5' and 3' of a selected nucleic acid construct that is present in another vector using standard ligation techniques. AAV vectors which contain ITRs have been described in, e. g., U. S. Patent no. 5,139, 941. In particular, several AAV vectors are available from the American Type Culture Collection ("ATCC") under Accession Numbers 53222, 53223, 53224, 53225 and 53226. Additionally, chimeric genes can be produced synthetically to include AAV ITR sequences arranged 5' and 3' of one or more selected nucleic acid sequences. Preferred codons for expression of the chimeric gene sequence in mammalian CNS cells can be used. The complete chimeric sequence is assembled from overlapping oligonucleotides prepared by standard methods. In order to produce AAV virions, an AAV expression vector is introduced into a suitable host cell using known techniques, such as by transfection. A number of transfection techniques are generally known in the art. See, e. g. Sambrook et al. (1989): Molecular Cloning, a laboratory manual, Cold Spring Harbor Laboratories, New York; Davis et al. (1986): Basic Methods in Molecular Biology, Elsevier. Particularly suitable transfection methods include calcium phosphate co-precipitation, direct microinjection into cultured cells, electroporation, liposome mediated gene transfer, lipid-mediated transduction and nucleic acid delivery using high-velocity microprojectiles.

### Agents related to the peptides of the invention

A fourth object ot the invention is an agent which, by displaying binding activity to peptides of sequence SEQ ID NO:2, SEQ ID NO:3 or SEQ ID NO:4, impedes a proper LRRK2/PP1 interaction. Said compound can be, for example, an aptamer or an antibody directed against said peptides of sequence SEQ ID NO:2, SEQ ID NO:3 or SEQ ID NO:4 or a conservative variant thereof.

The terms "compound" or "agent" are used herein indifferently to designate the peptides or agents inhibitors of LRRK2/PP1 interaction.

Consequently, in some embodiments, the aptamer or antibody of the present invention specifically binds to a peptide consisting of a f peptide of sequence SEQ ID NO:2, SEQ ID NO:3 or SEQ ID NO:4, or any conservative variant thereof thereby resulting inhibiting LRRK2/PP1 interaction.

The term "antibody" is thus used to refer to any antibody-like molecule that has an antigen binding region, and this term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991, specifically incorporated herein by reference). Diabodies, in particular, are further described in EP 404097 and WO 93/11 161, whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art.

In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

The term "Fab" denotes an antibody fragment having a molecular weight of about 50,000 Da and antigen binding activity, in which about a half of the N-terminal side of H chain and the entire L chain, among fragments obtained by treating IgG with a protease, papain, are bound together through a disulfide bond.

The term "F(ab')2" refers to an antibody fragment having a molecular weight of about 100,000 Da and antigen binding activity, which is slightly larger than the Fab bound via a disulfide bond of the hinge region, among fragments obtained by treating IgG with a protease, pepsin.

The term "Fab'" refers to an antibody fragment having a molecular weight of about 50,000 Da and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

Monoclonal antibodies may be generated using the method of Kohler and Milstein (1975). To prepare monoclonal antibodies useful in the invention, a mouse or other appropriate host animal is immunized at suitable intervals (e.g., twice-weekly, weekly, twice-monthly or monthly) with the appropriate antigenic forms (i.e. polypeptides of the present invention). The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes.

Briefly, the recombinant polypeptide of the invention may be provided by expression with recombinant cell lines. Recombinant forms of the polypeptides may be provided using any previously described method. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods. Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

Significantly, as is well-known in the art, only a small portion of an antibody molecule, the paratope, is involved in the binding of the antibody to its epitope (see, in general, Clark, W. R. (1986) The Experimental Foundations of Modern Immunology Wiley & Sons, Inc., New York; Roitt, I. (1991) Essential Immunology, 7th Ed., Blackwell Scientific Publications, Oxford). The Fc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDRS). The CDRs, and in particular the CDRS regions, and more particularly the heavy chain CDRS, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody.

In some embodiments, the antibody is a humanized antibody. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567, 5,225,539, 5,585,089, 5,693,761, 5,693,762 and 5,859,205, which are hereby incorporated by reference. The above U.S. Pat. Nos. 5,585,089 and 5,693,761, and WO 90/07861 also propose four possible criteria which may be used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3A of the CDRs in a three dimensional model of the antibody and is predicted to be capable of interacting with the CDRs. The above methods are merely illustrative of some of the methods that one skilled in the art could employ to make humanized antibodies. One of ordinary skill in the art will be familiar with other methods for antibody humanization.

In some embodiments, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgGI, IgG2, IgG3, IgG4, IgA and IgM molecules. A "humanized" antibody retains a similar antigenic specificity as the original antibody. However, using certain methods of humanization, the affinity and/or specificity of binding of the antibody may be increased using methods of "directed evolution", as described by Wu et al., I. Mol. Biol. 294: 151, 1999, the contents of which are incorporated herein by reference.

Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein, the contents of which are incorporated herein by reference. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans. *In vitro* methods also exist for producing human antibodies. These include phage display technology (U.S. Pat. Nos. 5,565,332 and 5,573,905) and *in vitro* stimulation of human B cells (U.S. Pat. Nos. 5,229,275 and 5,567,610). The contents of these patents are incorporated herein by reference.

Thus, as will be apparent to one of ordinary skill in the art, the present invention also provides for F(ab')2, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDRI and/or CDR2 and/or light chain CDR3 regions have been replaced by homologous human or non-human sequences; chimeric Fab fragment antibodies in which the FR and/or CDRI and/or CDR2 and/or light chain CDR3 regions have been replaced by homologous human or non-human sequences; and chimeric Fd fragment antibodies in which the FR and/or CDRI and/or CDR2 regions have been replaced by homologous human or non-human sequences. The present invention also includes so-called single chain antibodies.

The various antibody molecules and fragments may derive from any of the commonly known immunoglobulin classes, including but not limited to IgA, secretory IgA, IgE, IgG and IgM. IgG subclasses are also well known to those in the art and include but are not limited to human IgGI, IgG2, IgG3 and IgG4.

Aptamers are a class of molecule that represents an alternative to antibodies in term of molecular recognition. Aptamers are oligonucleotide sequences with the capacity to recognize virtually any class of target molecules with high affinity and specificity. Such ligands may be isolated through Systematic Evolution of Ligands by Exponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods (Colas et al., 1996).

### Therapeutic methods and uses of the invention:

As used herein, "treatment" includes the therapy, prevention, prophylaxis, retardation or reduction of symptoms provoked by or of the causes of a disease. When related to a neurodegenerative disease, said neurodegenerative disease is, for example selected from tauopathies like Alzheimer's disease (AD), Frontotemporal dementia Primary age-related tauopathy (PART)/Neurofibrillary tangle-predominant senile dementia, Chronic traumatic encephalopathy (CTE), Progressive supranuclear palsy, Corticobasal degeneration, parkinsonism linked to chromosome 17, Lytico-Bodig disease (Parkinson-dementia complex of Guam), Ganglioglioma and gangliocytoma, Meningioangiomatosis, Postencephalitic parkinsonism, Subacute sclerosing panencephalitis, lead encephalopathy, tuberous sclerosis, Pantothenate kinase-associated neurodegeneration, Fronto temporal dementia and lipofuscinosis. Said neurodegenerative disease is selected from α-synucleopathies like Parkinson's disease (PD), dementia with Lewy bodies (DLB), and multiple system atrophy (MSA). In a particular embodiment, said α-synucleopathy is PD.

When related to a neurodegenerative disease, the term "treatment" particularly includes the maintenance or the protection neuronal processes like neurite outgrowth and synaptic plasticity in the treated subjects.

When related to PD, the term "treatment" includes in particular the control of disease progression and associated motor and non-motor symptoms.

LRRK2 is known in the art to be implicated in numerous cellular processes. Peptides of the invention as well as agents "Inhibitors of LRRK2/PP1 interaction" as described above are efficient in altering interaction of LRRK2/PP1 and thereby in altering phosphorylation and localisation of LRRK2. Further as shown in the experimental section, peptides inhibitors of LRRK2/PP1 interaction of the invention efficiently cross BBB and target brain neuronal cells thanks to the shuttle moiety of these peptides. Without to be bound by any theory they are thought to interfere with the already formed LRRK2/PP1 complexes and or to impede their formation, without altering otherwise normal functions of these proteins. Indeed, no toxicity is detected for the animals administered with these peptides.

In this regard in an embodiment this invention relates to a peptide and/or agent "inhibitor of LRRK2/PP1 interaction" of the invention, or salts or prodrugs or derivatives of any purity or sustained release formulations thereof, for use as a medicament.

In a preferred embodiment, this invention relates to a composition comprising at least one peptide and/or agent "inhibitor of LRRK2/PP1 interaction" of the invention, or salts or prodrugs or derivatives of any purity or sustained release formulations thereof, for use in the treatment of a neurodegenerative disease. In a more preferred embodiment this invention relates to a composition comprising at least one peptide or agent of the invention, or salts or prodrugs or derivatives of any purity or sustained release formulations thereof, for use in the treatment of a disease selected from tauopathies like Alzheimer's disease (AD), frontotemporal dementia Primary age-related tauopathy (PART)/neurofibrillary tangle-predominant senile dementia, Chronic Traumatic Encephalopathy (CTE), progressive supranuclear palsy, corticobasal degeneration, parkinsonism linked to chromosome 17, Lytico-Bodig disease (Parkinson-dementia complex of Guam), ganglioglioma and gangliocytoma, meningioangiomatosis, postencephalitic parkinsonism, subacute sclerosing panencephalitis, lead encephalopathy, tuberous sclerosis, pantothenate kinase-associated neurodegeneration, fronto temporal dementia and lipofuscinosis. In another preferred embodiment this invention relates to a composition comprising a peptide or agent of the invention, or salts or prodrugs or derivatives of any purity or sustained release formulations thereof, for use in the treatment an α-synucleopathy like Parkinson's disease (PD), dementia with Lewy bodies (DLB), and multiple system atrophy (MSA). In a particularly preferred embodiment, this invention relates to a composition comprising at least one peptide or an agent of the invention, or salts or prodrugs or derivatives of any purity or sustained release formulations thereof, for use in the treatment of PD.

Said at least peptide of said composition is selected among the peptides described above of structure Nt-Cargo-Linker-Shuttle-Ct or of sequence peptides of sequence SEQ ID NO:2, SEQ ID NO:3 or SEQ ID NO:4 or any conservative variant thereof.

In an embodiment, the invention relates to a composition comprising at least one peptide selected from :
- SEQ ID NO:2 : VQGSRPGKNVQLTENE,
- SEQ ID NO:3 : AEVVAKFLHKHDLDLICRAH, or
- SEQ ID NO:4 : NPGGRPITPPRNSAKAKK,
or a conservative variant thereof for use in treating a neurodegenerative disease, preferably an α-synucleopathy selected from PD, MSA or DLB.

In an embodiment, the invention relates to a composition comprising at least one peptide inhibitor of LRRK2/PP1 interaction of structure Nt -Cargo - Linker - Shuttle-Ct as exposed above, or a conservative variant thereof.

In a particular embodiment, the invention relates to a composition comprising at least one peptide inhibitor of LRRK2/PP1 interaction of structure Nt -Cargo - Linker - Shuttle- Ct as described above wherein cargo moiety is selected from :
- SEQ ID NO:2 : VQGSRPGKNVQLTENE,
- SEQ ID NO:3 : AEVVAKFLHKHDLDLICRAH,
- SEQ ID NO:4 : NPGGRPITPPRNSAKAKK,
- SEQ ID NO:7 : INRLLEISPY,
- SEQ ID NO:8 : LINRLLEISPY,
- SEQ ID NO:9 : SRLINRLLEISPY,
- SEQ ID NO:10 : PMGFWSRLI,
- SEQ ID NO:11 : GFWSRLINRLLEISPY,
- SEQ ID NO:12 : PMGFWSRLINRLLEISPY,
- SEQ ID NO:13 : GFWSRLINRLLEI, or
- SEQ ID NO:14 : PMGFWSRLINRLLEI,
or a conservative variant thereof.

Any linker that provide sufficient space and flexibility between the cargo and the shuttle moiety provided that it is not too big is suitable for being used in the peptide of the invention. In a particular embodiment, the invention relates to a composition comprising at least one peptide inhibitor of LRRK2/PP1 interaction of structure Nt - Cargo - Linker - Shuttle- Ct as described above wherein Linker moiety is a PEG derivative moiety or another suitable small molecule, for example selected from : Amino-PEG4-alcohol, Amino-PEG6-alcohol, Amino-PEG8-alcohol, Amino-PEG12-alcohol, Amino-PEG8-acid, Amino-PEG12-acid, Amino-PEG24-acid, or 6-aminohexanoic acid. In another embodiment, the invention relates to a composition comprising at least one peptide inhibitor of LRRK2/PP1 interaction of structure Nt - Cargo - Linker - Shuttle- Ct as described above wherein Linker moiety is a di or tripeptide, for example selected from GGS (SEQ ID NO:6), GGG (SEQ ID NO:33), AAN (SEQ ID NO:34), GG (SEQ ID NO:35), FK (SEQ ID NO:36), VA (SEQ ID NO:37), VV (SEQ ID NO:38), VG (SEQ ID NO:39).

In a more particular embodiment of said composition according to the invention the linker moiety in said at least one peptide inhibitor of LRRK2/PP1 interaction of structure Nt -Cargo - Linker - Shuttle- Ct is selected peptide of SEQ ID NO:6 : GGS or 6-aminohexanoic acid.

In an even more particular embodiment the invention relates to a composition comprising at least one peptide inhibitor of LRRK2/PP1 interaction of structure Nt - Cargo - Linker - Shuttle- Ct, wherein :
- Shuttle moiety is of SEQ ID NO:1 or conservative variant thereof,
- Linker moiety is 6-aminohexanoic acid, and
- Cargo moiety is selected from :
   - SEQ ID NO:2 : VQGSRPGKNVQLTENE,
   - SEQ ID NO:3 : AEVVAKFLHKHDLDLICRAH,
   - SEQ ID NO:4 : NPGGRPITPPRNSAKAKK,
   - SEQ ID NO:7 : INRLLEISPY,
   - SEQ ID NO:8 : LINRLLEISPY,
   - SEQ ID NO:9 : SRLINRLLEISPY,
   - SEQ ID NO:10 : PMGFWSRLI,
   - SEQ ID NO:11 : GFWSRLINRLLEISPY,
   - SEQ ID NO:12 : PMGFWSRLINRLLEISPY,
   - SEQ ID NO:13 : GFWSRLINRLLEI, or
   - SEQ ID NO:14 : PMGFWSRLINRLLEI, or a conservative variant thereof,
for use in treating a neurodegenerative disease, preferably an α-synucleopathy selected from PD, MSA or DLB.

In a preferred embodiment, the invention relates to a composition comprising at least one peptide inhibitor of LRRK2/PP1 interaction of structure Nt -Cargo - Linker - Shuttle- Ct, wherein the shuttle moiety is peptide of SEQ ID NO:1, the linker is 6-aminohexanoic acid and the cargo moiety is of sequence SEQ ID NO:13.

In an even more particular embodiment the invention relates to a composition comprising at least one peptide inhibitor of LRRK2/PP1 interaction of structure Nt - Cargo - Linker - Shuttle- Ct, wherein :
- Shuttle moiety is of SEQ ID NO:1 or conservative variant thereof,
- Linker moiety is of SEQ ID NO:6, and
- Cargo moiety is selected from :
   - SEQ ID NO:2 : VQGSRPGKNVQLTENE,
   - SEQ ID NO:3 : AEVVAKFLHKHDLDLICRAH,
   - SEQ ID NO:4 : NPGGRPITPPRNSAKAKK,
   - SEQ ID NO:7 : INRLLEISPY,
   - SEQ ID NO:8 : LINRLLEISPY,
   - SEQ ID NO:9 : SRLINRLLEISPY,
   - SEQ ID NO:10 : PMGFWSRLI,
   - SEQ ID NO:11 : GFWSRLINRLLEISPY,
   - SEQ ID NO:12 : PMGFWSRLINRLLEISPY,
   - SEQ ID NO:13 : GFWSRLINRLLEI, or
   - SEQ ID NO:14 : PMGFWSRLINRLLEI, or a conservative variant thereof,
for use in treating a neurodegenerative disease, preferably an α-synucleopathy selected from PD, MSA or DLB.

In another embodiment, a composition of the invention comprises at least one agent inhibitor of LRRK2/PP1 interaction selected from an aptamer or an antibody as described above.

A composition according to the invention typically comprises one or several pharmaceutically acceptable carriers or excipients. Also, for use in the present invention, compounds of the invention are usually mixed with pharmaceutically acceptable excipients or carriers. In this regard, in a particular embodiment a composition according to the invention is a pharmaceutical composition comprising said peptide and/or agent inhibitor of LRRK2/PP1 interaction as exposed above.

In another preferred embodiment, the invention relates to a method of treating a disease selected from tauopathies like Alzheimer's disease (AD), frontotemporal dementia Primary age-related tauopathy (PART)/neurofibrillary tangle-predominant senile dementia, Chronic Traumatic Encephalopathy (CTE), progressive supranuclear palsy, corticobasal degeneration, parkinsonism linked to chromosome 17, Lytico-Bodig disease (Parkinson-dementia complex of Guam), ganglioglioma and gangliocytoma, meningioangiomatosis, postencephalitic parkinsonism, subacute sclerosing panencephalitis, lead encephalopathy, tuberous sclerosis, pantothenate kinase-associated neurodegeneration, fronto temporal dementia and lipofuscinosis, or α-synucleopathies like Parkinson's disease (PD), dementia with Lewy bodies (DLB), or multiple system atrophy (MSA), comprising administering a peptide and/ or an agent inhibitor of LRRK2/PP1 of the invention as exposed above.

More particularly, said method comprises administering at least one peptide inhibitor of LRRK2/PP1 interaction of structure Nt -Cargo - Linker - Shuttle- Ct, wherein :
- Shuttle moiety is of SEQ ID NO:1 or conservative variant thereof,
- Linker moiety is 6-aminohexanoic acid, and
- Cargo moiety is selected from :
   - SEQ ID NO:2 : VQGSRPGKNVQLTENE,
   - SEQ ID NO:3 : AEVVAKFLHKHDLDLICRAH,
   - SEQ ID NO:4 : NPGGRPITPPRNSAKAKK,
   - SEQ ID NO:7 : INRLLEISPY,
   - SEQ ID NO:8 : LINRLLEISPY,
   - SEQ ID NO:9 : SRLINRLLEISPY,
   - SEQ ID NO:10 : PMGFWSRLI,
   - SEQ ID NO:11 : GFWSRLINRLLEISPY,
   - SEQ ID NO:12 : PMGFWSRLINRLLEISPY,
   - SEQ ID NO:13 : GFWSRLINRLLEI, or
   - SEQ ID NO:14 : PMGFWSRLINRLLEI, or a conservative variant thereof.

In a preferred embodiment, said method comprises administering at least one peptide inhibitor of LRRK2/PP1 interaction of structure Nt -Cargo - Linker - Shuttle- Ct wherein the shuttle moiety is peptide of SEQ ID NO:1, the linker is 6-aminohexanoic acid and the cargo moiety is of sequence SEQ ID NO:13.

More particularly, said method comprises administering at least one peptide inhibitor of LRRK2/PP1 interaction of structure Nt -Cargo - Linker - Shuttle- Ct, wherein :
- Shuttle moiety is of SEQ ID NO:1 or conservative variant thereof,
- Linker moiety is of SEQ ID NO:6, and
- Cargo moiety is selected from :
   - SEQ ID NO:2 : VQGSRPGKNVQLTENE,
   - SEQ ID NO:3 : AEVVAKFLHKHDLDLICRAH,
   - SEQ ID NO:4 : NPGGRPITPPRNSAKAKK,
   - SEQ ID NO:7 : INRLLEISPY,
   - SEQ ID NO:8 : LINRLLEISPY,
   - SEQ ID NO:9 : SRLINRLLEISPY,
   - SEQ ID NO:10 : PMGFWSRLI,
   - SEQ ID NO:11 : GFWSRLINRLLEISPY,
   - SEQ ID NO:12 : PMGFWSRLINRLLEISPY,
   - SEQ ID NO:13 : GFWSRLINRLLEI, or
   - SEQ ID NO:14 : PMGFWSRLINRLLEI, or a conservative variant thereof.

In a particular embodiment, said method comprises administering at least one peptide selected from :
- SEQ ID NO:2 : VQGSRPGKNVQLTENE,
- SEQ ID NO:3 : AEVVAKFLHKHDLDLICRAH, or
- SEQ ID NO:4 : NPGGRPITPPRNSAKAKK,
or a conservative variant thereof.

In a preferred embodiment, the above methods, or compositions can be used in a subject suffering from or who is at risk of developing a neurodegenerative disease as listed above or symptoms associated with said disease.

In a preferred embodiment, the above methods, or compositions can be used in a subject suffering from or who is at risk of developing PD disease or symptoms associated with PD.

The combination of early detection of non-motor symptoms, most particularly anosmia, with imaging techniques (Single-photon emission computed technology, Positron Emission Tomography) to assess changes in striatal dopamine transporter may be a suitable approach to identify at risk PD patients prior to the appearance of motor symptoms, thus allowing early start of neuroprotective therapy using the compounds, compositions or therapies according to the invention.

Some PD cases can be attributed to mutations within genes such as SNCA (alpha-synuclein), PRKN (parkin), LRRK2 (leucine-rich repeat kinase 2), PINK1 (PTEN-induced putative kinase 1), DJ-1 and ATP13A2 and eleven gene loci (PARK1-PARK11). In this regard, in a particular embodiment, the invention relates to the use of the above methods, compositions or therapies for the treatment of PD in a subject having a mutation in at least one of the following genes: SNCA, PRKN, LRRK2, PINK1, DJ-1, ATP13A2 and PARK1 to PARK11.

High concentrations exposure or chronic exposure to metals such as manganese, copper or leads, or chemicals, such as pesticides (e.g. paraquat, rotenone and maneb), are likely to cause PD or related disorders. In this regard, in a particular embodiment, the invention relates to the use of the above methods, compositions or therapies in the treatment of PD or related disorders, in a subject exposed, suspected to have been exposed or at risk of be exposed, to chemicals or metals known to be risk factors for developing PD or related disorders.

The above methods, compositions or therapies may further be used in conjunction or association or combination with additional drugs or treatments.

In a particular embodiment, additional therapies used in conjunction with compositions or compounds for use in treating PD according to the present invention, may comprise one or more drug(s) that ameliorate symptoms of PD, one or more drug(s) that could be used for palliative treatment of PD or one or more drug(s) currently evaluated in the frame of clinical trials for treating of PD. Therefore, compositions of the invention can be combined with dopaminergic drugs such as dopamine precursors (preferably levodopa), dopamine receptor agonists (preferably pergolide, cabergoline, lisuride, pramipexole, ropinirole or apomorphine) or inhibitors of dopamine-metabolizing enzymes (preferably selegiline, rasagiline, tolcapone or entacapone). Compositions of the invention can also be combined with treatment of the non-motor symptoms of PD, preferably Clozapine, Desipramine, Citalopram, Nortriptyline, Paroxetine, Atomoxetine, Venlafaxine, Amantadine, Donepezil, Rivastigmine or Memantine.

In this regard, in a further embodiment this invention relates to a composition, for use in the treatment of PD, comprising a composition as defined above, in combination with at least one compound selected from the group consisting of levodopa, pergolide, cabergoline, lisuride, pramipexole, ropinirole, apomorphine, selegiline, rasagiline, tolcapone, entacapone, clozapine, desipramine, citalopram, nortriptyline, paroxetine, atomoxetine, venlafaxine, amantadine, donepezil, rivastigmine and memantine, or salts or prodrugs or derivatives of any purity or sustained release formulations thereof.

In a particular embodiment, when combination therapies of the invention comprise dopamine precursor, they can be further combined with at least one compound selected from peripheral dopa decarboxylase inhibitors or catechol-O-methyl transferase inhibitors. More particularly, when combination therapies of the invention comprise a dopamine precursor, they can be further combined with at least one compound selected from carbidopa, benserazide or entacapone.

In another embodiment, compositions or combination therapies of the invention can be used in conjunction with surgical therapy for PD such as deep brain stimulation. More particularly, surgical therapies are deep brain stimulation of the subthalamic nucleus or of the globus pallidus interna.

In this regard, the invention also relates to a composition as defined above, for use in combination with deep brain stimulation of the subthalamic nucleus or of the globus pallidus interna, in the treatment of PD and related disorders.

Therapy according to the invention may be provided at home, the doctor's office, a clinic, a hospital's outpatient department, or a hospital, so that the doctor can observe the therapy's effects closely and make any adjustments that are needed.

The duration of the therapy depends on the stage of the disease being treated, age and condition of the patient, and how the patient responds to the treatment. When the therapy is combinatorial, the dosage, frequency and mode of administration of each component of the combination can be controlled independently. For example, one compound may be administered orally while the second may be administered intramuscularly. Combination therapy may be given in on-and-off cycles that include rest periods so that the patient's body has a chance to recovery from side-effects. The compounds may also be formulated together such that one administration delivers all compounds.

Possible pharmaceutical compositions include those suitable for oral, rectal, topical (including transdermal, buccal and sublingual), parenteral (including subcutaneous, intramuscular, intravenous and intradermal), or intrathecal administration. More commonly these pharmaceutical formulations are prescribed to the patient in "patient packs" containing a number dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical formulation, as herein before described, in combination with packaging material suitable for said formulations. In such a patient pack the intended use of a formulation for the treatment can be inferred by instructions, facilities, provisions, adaptations and/or other means to help using the formulation most suitably for the treatment. Such measures make a patient pack specifically suitable for and adapted for use for treatment with the compounds of the present invention.

The peptide or agent inhibitor of LRRK2/PP1 interaction of the invention may be contained, in any appropriate amount, in any suitable carrier substance. It may be present in an amount of up to 99% by weight of the total weight of the composition. The pharmaceutical composition may be provided in a dosage form that is suitable for the oral, parenteral (e.g., intravenously, intramuscularly), rectal, cutaneous, nasal, inhalant, skin (patch), intrathecal or ocular administration route. Thus, the composition may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols.

Typically, the peptide or agent inhibitor of LRRK2/PP1 interaction of the invention of the invention as described above is administered to the subject in a therapeutically effective amount.

By a "therapeutically effective amount" of the peptide or agent inhibitor of LRRK2/PP1 interaction of the present invention as above described is meant a sufficient amount of the compound. It will be understood, however, that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the agent of the present invention for the symptomatic adjustment of the dosage to the subject to be treated. A medicament typically contains from about 0.01 mg to about 500 mg of the peptide or agent inhibitor of LRRK2/PP1 interaction of the present invention of the present invention, preferably from 1 mg to about 100 mg of the agent of the present invention. An effective amount of the drug is ordinarily supplied at a dosage level from 0.0002 mg/kg to about 20 mg/kg of body weight per day, especially from about 0.001 mg/kg to 7 mg/kg of body weight per day. For the reasons given above, these dosage ranges are intended to provide general guidance and support for the teachings herein but are not intended to limit the scope of the invention.

The following examples are given for purposes of illustration and not by way of limitation.

### EXAMPLES

### 1. MATERIALS AND METHODS

### Peptides synthesis and sequence

Peptides were synthesized in an automated multiple peptide synthesizer with solid phase procedure and standard Fmoc chemistry. The purity and composition of the peptides was confirmed by reverse phase HPLC and mass spectrometry (Frank and Overwin, 1996, Methods in Molecular Biology).

### Fluorescence live imaging

The 6-8 weeks old female CD1 mice were purchased from Charles River Laboratories (L'Arbresle, France). All mice were maintained under conditions and protocols in accordance with the Directive 2010/63/UE of the Council of Europe on Animal Welfare. The study (number authorization 27656) was approved by the French Ethics Committee for animal experimentation number 74 and all experiments were conducted following the guidelines of the aforementioned committee.

Animals were sedated with 2% isoflurane in air. Mice were injected into the retro-orbital vein with the peptides labelled with the fluorochrome Cy7 followed by fluorescence imaging at different times upon injection. Acquisitions were performed 15 min after IV injection, then regularly, up to 6 h post-administration. Before the acquisition, the animal was anesthetized by isoflurane inhalation (2% air-isoflurane blend). Then, the mouse was placed inside the acquisition chamber of an *in vivo* imaging system equipped with a cooled slow-scan CCD camera and driven with Indigo software (Berthold Technologies, Bad Wildbad, Germany). Images were captured at 1 × 1 binning with exposure times ranging from 0.1 to 1.0 s, depending on the fluorescence intensity. After the *in vivo* imaging, mice were scarified, and the brain isolated for *in vivo* imaging.

### Immunochemistry - labelling with peptides of the invention

The immunostaining was procedure was performed on formalin-fixed deparaffinized 3 µm thick sections of CD1 mice embryo stained with Cy5-labelled peptide 1 (100 µM) using Venture platform (Roche Diagnostic) according to the manufacturer protocol. Samples were visualized by fluorescence microscopy.

### Measurement of toxicity of peptides

Eight weeks old female CD1 mice were intraperitoneally injected with 5mg/Kg of peptide or saline (control), 5 days per week. The weight of the animals was followed for 2 weeks. No symptoms of toxicity were observed (diarrhoea, loss of wight, fiver etc.).

### Analysis of peptide stability in human serum

The peptides were incubated at 37°C in 250 µL of human serum (Gibco) for different periods of time. Samples were collected and peptide degradation stopped by freezing at -20°C. The peptide was extracted from samples using the Proteo Miner Protein Enrichment System (Bio-Rad). The percentage of intact peptide was estimated by MS using MALDI-TOFF using the protocol previously described (Bruker Autoflex II) according to the manufacturer's instructions. Measurements were performed in triplicate. MS data were analysed using the software Cliprot tools, Flex analysis, Bruker.

### Immunoprecipitation, western blot and in vitro protein/protein interaction competition

Cells (5×10⁶) were lysed for 20 min at 4°C in lysis buffer (50 mM Tris pH8, 1% NP40, 137 mM NaCl, 1 mM MgCl2, 1 mM CaCl2, 10% glycerol and protease inhibitor mixture Sigma Aldrich). Lysates (500 µg) were immunoprecipitated with the appropriated antibody overnight at 4 °C and protein A/G Sepharose was added for 1h at 4°C. After washing with 1x TBST (20 mM Tris-HCl pH7.5, 150 mM NaCl, 0.05% Tween 20), the PP1/LRRK2 interaction was competed using 1 mM of the peptides for 30 min at room temperature. After several washing steps, immunoprecipitates were separated by SDS-PAGE, transferred to nitrocellulose and blotted with anti PP1 antibody. The membrane was washed and incubated with PO-conjugates secondary antibody. Protein detection was performed using the ECL system. As internal control, the blot was also hybridized with anti-LRRK2 antibody. Western blots were densitometred using Image J.

### Assay of the peptide of the invention as a preventive or curative treatment in a model of a-synucleopathy

AAV vector-mediated delivery of alpha-synuclein (human, wild type) are chosen to assay the protective or curative effect of the peptide of the invention. This model is a well-known model for more than 10 years now (Koprich, et al. 2010). Briefly, CD1 mice are injected with peptide 1 (table 1, 5 mg/kg or 10 mg/kg) or saline during 4 weeks, starting from either 2(preventive assay) or 4 weeks from initiation of first symptoms. At 3 and 6 months motor symptoms are tested and mice are euthanized and their brain harvested for histological analysis and assessment of neuronal degeneration.

### 2. RESULTS

Peptides tested and discussed in the experimental section are referenced in table 1 below:

**Table 1**

| **Peptide number** | **Peptides discussed in the experimental section** | **SEQ ID** |
|---|---|---|
| **1** | GFWSRLINRLLEI- AHx -SRRVISRAKLAAAL | SEQ ID NO:15 |
| **2** | GFWSRLINRLLEI- GGS -SRRVISRAKLAAAL | SEQ ID NO:16 |
| **3** | SRRVISRAKLAAAL- AHx -GFWSRLINRLLEI | SEQ ID NO:17 |
| **4** | SRRVISRAKLAAAL- GGS -GFWSRLINRLLEI | SEQ ID NO:18 |
| **5** | VQGSRPGKNVQLTENE- AHx-SRRVISRAKLAAAL | SEQ ID NO:19 |
| **6** | AEVVAKFLHKHDLDLICRAH- AHx- SRRVISRAKLAAAL | SEQ ID NO:20 |
| **7** | NPGGRPITPPRNSAKAKK- AHx-SRRVISRAKLAAAL | SEQ ID NO:21 |
| **8** | INRLLEISPY- AHx-SRRVISRAKLAAAL | SEQ ID NO:22 |
| **9** | LINRLLEISPY- AHx-SRRVISRAKLAAAL | SEQ ID NO:23 |
| **10** | PMGFWSRLI- AHx-SRRVISRAKLAAAL | SEQ ID NO:24 |
| **11** | GFWSRLINRLLEISPY- AHx-SRRVISRAKLAAAL | SEQ ID NO:25 |
| **12** | PMGFWSRLINRLLEI- AHx-SRRVISRAKLAAAL | SEQ ID NO:26 |
| **13** | VQGSRPGKNVQLTENE- AHx-SRRVISRAKLAAAL | SEQ ID NO:27 |
| **14** | AEVVAKFLHKHDLDLICRAH- AHx- SRRVISRAKLAAAL | SEQ ID NO:28 |
| **15** | NPGGRPITPPRNSAKAKK- AHx-SRRVISRAKLAAAL | SEQ ID NO:29 |
| **16** | GFWSRLlNRLLEI- AHx -TFFYGGSRGKRNNFKTEEY | SEQ ID NO:30 |
| **17** | TFFYGGSRGKRNNFKTEEY- GGS - GFWSRLINRLLEI | SEQ ID NO:31 |
| **18** | GFWSRLINRLLEI- AHx -AKRGARSTA | SEQ ID NO:32 |
| **19** | VQGSRPGKNVQLTENE | SEQ ID NO:2 |
| **20** | AEVVAKFLHKHDLDLICRAH | SEQ ID NO:3 |
| **21** | NPGGRPITPPRNSAKAKK | SEQ ID NO:4 |

### Identification of PP1 sequences involved in LRRK2/PP1 interaction - in vitro interaction testing for peptides of the invention

Overlapping dodecapeptides from PP1 protein were generated from PP1 protein sequence (SEQ ID NO:17) and immobilized on a cellulose membrane which was then hybridized with PP1 protein as exposed in material and method section. Among the sets of spots inventors selected 3 sets corresponding to 3 regions of PP1. Minimal peptides 19, 20, 21 were identified as interaction region of PP1 with LRRK2. Different peptides have been delineated in order to minimize the entropic cost upon peptide binding.

Said peptides were then tested to their ability to target the *in vitro* interaction LRRK2/PP1. This was tested by a competition assay using lysates from MDA-MB231 cell line, that was immunoprecipitated with anti-PP1 antibody and the interaction with LRRK2 was competed using the peptides. As shown in Figure 9, peptides are able to inhibit LRRK2/PP1 interaction: LRRK2 is detected in control PP1 immunoprecipitates, while it was not detected after competition with 1 mM of peptide 19 according to the invention ; an inhibition of LRRK2/PP1 interaction is also observed for peptides 20 or 21 (not shown). PP1 was used as internal control showing similar level in all conditions. These results show that peptides located in the regions identified as interacting with LRRK2 protein, and more specifically peptides 19, 20, 21 specifically target the interaction between human LRRK2 and PP1. Of note, the interaction of PP1 with caspase 9 is not inhibited by peptides of the invention, thereby showing the specificity of their inhibitory activity for LRRK2/PP1 interaction (not shown).

### LRRK2/PP1 inhibition assay using peptides inhibitor of LRRK2/PP1 interaction of structure Nt -Cargo - Linker - Shuttle- Ct

Figure 8 presents immunoprecipitation assays for testing the inhibition of LRRK2/PP1 interaction by peptides of structure Nt -Cargo - Linker - Shuttle- Ct of the invention. Arrow points the band of LRRK2/PP1 complex as detected with LRRK2 antibodies. No band reacting with LRRK2 is observed in PP1 immunoprecipitates for sample incubated with peptides 1 or 2. Therefore, peptides of the invention of structure Nt -Cargo - Linker - Shuttle- Ct are effective in inhibiting LRRK2/PP1 interaction. Said peptides were then tested to their ability to target the in vitro interaction LRRK2/PP1. This was tested by a competition assay using lysates from

### Crossing of the Blood Brain Barrier of chimeric peptides of the invention

Results of fluorescence brain imaging are summarized in table 2.

**Table 2**

| Peptide number | Nter moiety | linker | Cter moiety | Cross BBB | Stability | toxicity |
|---|---|---|---|---|---|---|
| 1 | GFWSRLINRLLEI | AHx | SRRVISRAKLAAAL | ++ | ++ | NO |
| 2 | GFWSRLINRLLEI | GGS | SRRVISRAKLAAAL | ++ | ++ | NO |
| 3 | SRRVISRAKLAAAL | AHx | GFWSRLINRLLEI | - | ++ | NO |
| 4 | SRRVISRAKLAAAL | GGS | GFWSRLINRLLEI | - | ++ | NO |
| 5 | VQGSRPGKNVQLTENE | AHx | SRRVISRAKLAAAL | ++ | ++ | NO |
| 6 | AEVVAKFLHKHDLDLICRAH | AHx | SRRVISRAKLAAAL | ++ | ++ | NO |
| 7 | NPGGRPITPPRNSAKAKK | AHx | SRRVISRAKLAAAL | ++ | ++ | NO |
| 8 | INRLLEISPY | AHx | SRRVISRAKLAAAL | ++ | ++ | NO |
| 9 | LINRLLEISPY | AHx | SRRVISRAKLAAAL | ++ | ++ | NO |
| 10 | PMGFWSRLI | AHx | SRRVISRAKLAAAL | ++ | ++ | NO |
| 11 | GFWSRLINRLLEISPY | AHx | SRRVISRAKLAAAL | ++ | ++ | NO |
| 12 | PMGFWSRLINRLLEI | AHx | SRRVISRAKLAAAL | ++ | ++ | NO |
| 13 | VQGSRPGKNVQLTENE | AHx | SRRVISRAKLAAAL | ++ | ++ | NO |
| 14 | AEVVAKFLHKHDLDLICRAH | AHx | SRRVISRAKLAAAL | ++ | ++ | NO |
| 15 | NPGGRPITPPRNSAKAKK | AHx | SRRVISRAKLAAAL | ++ | ++ | NO |
| 16 | GFWSRLINRLLEI | AHx | TFFYGGSRGKRNNF KTEEY | ++ | ++ | na |
| 17 | TFFYGGSRGKRNNFKTEEY | GGS | GFWSRLINRLLEI | - | ++ | na |
| 18 | GFWSRLINRLLEI | AHx | AKRGARSTA | - | ++ | na |

| | | | | | | |
|---|---|---|---|---|---|---|
| AHx : 6-aminohexanoic acid | | | | | | |

Tested chimeric peptides were labelled with Sulfo-Cyanine7 (Cy7 at their N-terminal moiety). As shown in the Figures 1 and 2 and table 2 peptides of structure Nt - Cargo - Linker - Shuttle- Ct. efficiently cross the BBB of the injected animals. Labelling decrease quickly after the injection, and no fluorescence is detected in the animal 24 h after injection. Surprisingly, no fluorescence was detected in the brain for the tested peptides of structure Nt -Shuttle - Linker - Cargo- Ct as for example peptides 3 or 4 (table 2). Of note, also no fluorescence is detected in the brain of the animals injected with peptides Nt -Cargo - Linker - Shuttle- Ct wherein the shuttle is LinTT1 (Figure 3, table 2 peptide 18) a shuttle moiety that is specific of tumoral cells (Simón-Gracia et al, 2018). Fluorescence is also detected for positive control wherein angioprep-2 is used as a shuttle peptide in the structure Nt -Cargo - Linker - Shuttle- Ct (table 2, peptide 16), but not for peptide 17 (Nt -Shuttle - Linker - Cargo- Ct). Angiopep2 is a shuttle peptide that have been designated as a shuttle for crossing BBB. Of note, peptides of the invention with shuttle of SEQ ID NO:1, which is much shorter that Angiopep2 and which targets reelin, are also effective in passing BBB.

Data presented on Figure 4 for peptide 1 show that crossing of BBB by peptides inhibitors of LRRK2/PP1 interaction and which display of the structure Nt -Cargo - Linker - Shuttle- Ct is dose dependant.

### Targeting of neuronal cells

Immunochemistry labelling of mouse embryo with the inhibitors of LRRK2/PP1 interaction of the structure Nt -Cargo - Linker - Shuttle- Ct of the invention show an important labelling of the midbrain of the embryos (Figure 5). Of note labelling of brain neuronal cells is also detected for mice injected with the labelled peptide 3 (not shown). The same was observed when labelled peptide is administered IV to the mice: labelling is found in neuronal cells of the brain of the injected mice (not shown). Therefore, inhibitors of LRRK2/PP1 interaction effectively target neuronal cells in brain. The same is observed for all the tested inhibitors of LRRK2/PP1 interaction of the invention.

### Stability in serum

As illustrated on Figure 6 for peptides 1 and 2, the inhibitors of LRRK2/PP1 interaction of invention resist to the numerous proteases present in human serum. After 24h of incubation at 37°C, more than 90% of peptide are still present. This important stability is particularly advantageous especially when considering the use of said peptides as a medicament. Of note, the stability of peptides wherein AHx is used as a linker is particularly high, as exemplified in Figure 6 for peptide 1.

### Toxicity in animals

When tested for a daily intraperitoneal administration over 15 days, no adverse effects is reported in animals administered with the tested inhibitors of LRRK2/PP1 interaction of invention. Further as illustrated in Figure 7, no impact on animal weight is observed.

### 3. Conclusion

Inventors have identified new penetrating and interfering peptides inhibitors of LRRK2/PP1 interaction of a particular structure in respect to the known consensus of classic bi-functional peptides. These peptides are shown efficient in crossing the BBB in a dose dependant manner and to target neuronal cells. Further, these peptides are particularly resistant to serum protease and not toxic upon daily treatment.

### REFERENCES

Carter BJ. Adeno-associated virus vectors. Curr Opin Biotechnol. 1992 Oct;3(5):533-9. Clark, W. R. (1986) The Experimental Foundations of Modern Immunology Wiley & Sons, Inc., New York.
Clark, W. R. (1986) The Experimental Foundations of Modern Immunology Wiley & Sons, Inc., New York;
Colas P, Cohen B, Jessen T, Grishina I, McCoy J, Brent R. Genetic selection of peptide aptamers that recognize and inhibit cyclin-dependent kinase 2. Nature. 1996 Apr 11;380(6574):548-50.
Davidson BL, Stein CS, Heth JA, Martins I, Kotin RM, Derksen TA, Zabner J, Ghodsi A, Chiorini JA. Recombinant adeno-associated virus type 2, 4, and 5 vectors: transduction of variant cell types and regions in the mammalian central nervous system. Proc Natl Acad Sci USA. 2000 Mar 28;97(7):3428-32.
Davis M., Dibner D. and J. F. Battey (Editors) (1986): Basic Methods in Molecular Biology, Elsevier.
Frank R, Overwin H. SPOT synthesis. Epitope analysis with arrays of synthetic peptides prepared on cellulose membranes. Methods Mol Biol. 1996;66:149-69.
Kabat EA, Wu TT. Identical V region amino acid sequences and segments of sequences in antibodies of different specificities. Relative contributions of VH and VL genes, minigenes, and complementarity-determining regions to binding of antibody-combining sites. J Immunol. 1991 Sep 1;147(5):1709-19.
Köhler G & Milstein C. Continuous cultures of fused cells secreting antibody of predefined specificity. Nature 1975 256, pages495-497.
Koprich, J. B., Johnston, T. H., Reyes, M. G., Sun, X., Brotchie, J. M. (2010). Expression of human A53T alpha-synuclein in the rat substantia nigra using a novel AAV1/2 vector produces a rapidly evolving pathology with protein aggregation, dystrophic neurite architecture and nigrostriatal degeneration with potential to model the pat. Mol. Neurodegener. 5, 43.
Kotin RM. Prospects for the use of adeno-associated virus as a vector for human gene therapy. Hum Gene Ther. 1994 Jul;5(7):793-801.
Lebkowski JS, McNally MM, Okarma TB, Lerch LB. Adeno-associated virus: a vector system for efficient introduction and integration of DNA into a variety of mammalian cell types. Mol Cell Biol. 1988 Oct;8(10):3988-96.
MacLeod D, Dowman J, Hammond R, Leete T, Inoue K, Abeliovich A. The familial Parkinsonism gene LRRK2 regulates neurite process morphology. Neuron. 2006 Nov 22;52(4):587-93.
Miklossy J, Arai T, Guo JP, Klegeris A, Yu S, McGeer EG, McGeer PL. LRRK2 expression in normal and pathologic human brain and in human cell lines. J Neuropathol Exp Neurol. 2006 Oct;65(10):953-63.
Muzyczka N. Use of adeno-associated virus as a general transduction vector for mammalian cells. Curr Top Microbiol Immunol. 1992;158:97-129.
Roitt, I. (1991) Essential Immunology, 7th Ed., Blackwell Scientific Publications, Oxford. Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Shelling AN, Smith MG. Targeted integration of transfected and infected adeno-associated virus vectors containing the neomycin resistance gene. Gene Ther. 1994 May;1:165-9. Simón-Gracia L, Hunt H, Teesalu T. Peritoneal Carcinomatosis Targeting with Tumor Homing Peptides. Molecules. 2018 May 16;23(5):1190. doi: 10.3390/molecules23051190. PMID: 29772690; PMCID: PMC6100015.
Vincent KA, Moore GK, Haigwood NL. Replication and packaging of HIV envelope genes in a novel adeno-associated virus vector system. Vaccines 90 : 353-359.
Wu H, Nie Y, Huse WD, Watkins JD Humanization of a murine monoclonal antibody by simultaneous optimization of framework and CDR residues. J Mol Biol. 1999 Nov 19;294(1):151-62.
Zhou SZ, Cooper S, Kang LY, Ruggieri L, Heimfeld S, Srivastava A, Broxmeyer HE. Adeno-associated virus 2-mediated high efficiency gene transfer into immature and mature subsets of hematopoietic progenitor cells in human umbilical cord blood. J Exp Med. 1994 Jun 1;179(6):1867-75.
Zapata G, Ridgway JB, Mordenti J, Osaka G, Wong WL, Bennett GL, Carter P. Engineering linear F(ab')2 fragments for efficient production in Escherichia coli and enhanced antiproliferative activity. Protein Eng. 1995 Oct;8(10):1057-62.

## Claims

1. A peptide inhibitor of LRRK2/PP1 interaction of following structure:
Nt -Cargo - Linker - Shuttle- Ct, wherein :
- "Shuttle" moiety is defined as a peptide of sequence of SEQ ID NO:1,
- "Linker" moiety is selected from Amino-PEG4-alcohol, Amino-PEG6-alcohol, Amino-PEG8-alcohol, Amino-PEG12-alcohol, Amino-PEG8-acid, Amino-PEG12-acid, Amino-PEG24-acid, 6-aminohexanoic acid, SEQ ID NO:6, SEQ ID NO:33, SEQ ID NO:34, SEQ ID NO:35, SEQ ID NO:36, SEQ ID NO:37,SEQ IDNO:38, SEQ ID NO:39,
- "Cargo" moiety is selected from :
i) a peptide selected from a peptide of sequence of SEQ ID NO:2, SEQ ID NO:3 or SEQ ID NO:4, or a conservative variant thereof,
ii) a peptide of a sequence of at least 7 amino acids in the region ranging from the residue at position 1701 to the amino acid residue at position 1715 of SEQ ID NO:5, or a conservative variant thereof.

2. The peptide inhibitor according to claim 1 wherein the "Cargo" moiety is selected from : SEQ ID NO:7, SEQ ID NO:8, SEQ ID NO:9, SEQ ID NO:10, SEQ ID NO:11, SEQ ID NO:12, SEQ ID NO:13, SEQ ID NO:14 or a conservative variant thereof.

3. The peptide inhibitor according to any one of preceding claims wherein the "Linker" moiety is selected from the tripeptide of SEQ ID NO:6 or 6-aminohexanoic acid.

4. The peptide inhibitor according to any one of preceding claims wherein the "Linker" moiety is selected 6-aminohexanoic acid and the "Cargo" moiety is SEQ ID NO:13.

5. The peptide inhibitor according to any one of claims 1-3 wherein the "Linker" is the tripeptide of sequence SEQ ID NO:6.

6. A nucleic acid molecule encoding for the peptide of claim 5.

7. A vector which comprises the nucleic acid molecule of claim 6.

8. A host cell transformed with the nucleic acid molecule of claim 6 or 7.

9. An antibody or aptamer which specifically binds to a peptide selected from a peptide of sequence of SEQ ID NO:2, SEQ ID NO:3 or of SEQ ID NO:4.

10. An agent selected from the group consisting in the peptide inhibitor of LRRK2/PP1 interaction of claim 1, the nucleic acid of claim 6, the aptamer of claim 9 or the antibody of claim 9 for use as a medicament.

11. The agent for use of claim 10 for use in the treatment of a neurodegenerative disorder.

12. The agent for use of claim 10 wherein said neurodegenerative disorder is an a-synucleinopathy, preferably selected from Parkinson's disease (PD), dementia with Lewy bodies (DLB), and multiple system atrophy (MSA).

13. An isolated peptide inhibitor of LRRK2/PP1 comprising a peptide of sequence of SEQ ID NO:2, SEQ ID NO:3 or SEQ ID NO:4, or a conservative variant thereof.
